# EUROPEAN PATENT APPLICATION

(11) **EP 1 710 317 A2**
(43) Date of publication of application: **11.10.2006**
(21) Application number: 06117150.0
(22) Date of filing: 13.07.2006
(51) Int. Cl.: C12P 13/04, C12P 13/06, C12P 13/08

(54) **Method for producing L-threonine and L-homoserine**

(71) Applicant: DEGUSSA AG, 40474 Düsseldorf (DE)
(72) Inventor: The designation of the inventor has not yet been filed

(57) **Abstract**

The invention relates to a method for the production of L-threonine and L-homoserine comprising culturing recombinant microorganisms of Escherichia whereby the expression of one or more genes and open reading frames (orf's) are modulated.

## Description

Besides D,L-methionine and L-lysine L-threonine is an important amino acid for animal nutrition. It is produced by fermentation technology using genetically modified strains of Escherichia coli. A review on L-threonine metabolism and production was recently published by Debabov (Advances in Biochemical Engineering Vol.79, 113-136 (2003)).

Recently the fermentative production of L-homoserine has also attracted increasing interest.

A multitude of publications and patent applications demonstrate the economic significance of L-threonine production by Escherichia coli.

Numerous L-threonine producers are known in the art; e. g.:
Escherichia coli Kat 69.9 (WO 02/26993),
Escherichia coli TH 14.97 (WO 02/26993),
Escherichia coli TH 21.97 (WO 02/26993),

Escherichia coli VNIIgenetika MG442 (US-A-4,278,765),
Escherichia coli VNIIgenetika 472T23 (US-A-5,631,157),
Escherichia coli BKIIM B-3996 (US-A-5,175,107),
Escherichia coli BKIIM B-3996ΔtdhΔpckA/pVIC40
   (WO 02/29080),

Escherichia coli KCCM-10132 (WO 00/09660),
Escherichia coli KCCM-10133 WO 00/09661, and
Escherichia coli KCCM-10168 WO 01/14525.

It was found that modulation of expression of one or more of the following genes and open reading frames (orf's) of Escherichia, in particular Escherichia coli, thus providing modified strains, was beneficial for L-threonine as well as L-homoserine production using Escherichia coli:
a) one or more of the genes selected from the group consisting of nrdH gene, nrdI gene, nrdE gene and nrdF gene (Jordan et al, Molecular Microbiology 19(4), 777-790 (1996) and Vassinova et al, Microbiology 146 (12), 3171-3182 (2000));
b) ybaL, as shown in SEQ ID NO's:1, 2, 3, 4, 5 and 6;
c) ygaC, as shown in SEQ ID NO's:7, 8, 9 and 10;
d) yohJ, as shown in SEQ ID NO's:11, 12, 13 and 14;
e) yjgP, as shown in SEQ ID NO's:15, 16, 17 and 18; and
f) yggW, as shown in SEQ ID NO's:19, 20, 21 and 22.

It is obvious that orf's can be used which code for polypeptides which are at least 90%, preferred 95%, particularly preferred 99% identical to those included in the list of SEQ ID NO's:1 to 22.

In particular strains are used for said genetic modification measures, which already produce said amino acids, preferably L-threonine and L-homoserine. The term "produce" means that the unmodified strains used for the measures of the invention are able to excrete the desired amino acid into the fermentation medium.

In this context the term "modulation of expression" includes measures of enhancing expression, decreasing expression and eliminating expression of the polypeptides encoded by said genes and orf's.

Measures of enhancing expression include amplification of said genes and orf's using vectors, e. g. plasmid vectors like e. g. pSC101 and pBR322, use of strong promoters like e. g. T3, SP6, M13, lac, tac, λPL and λPR. Amplification can also be achieved by inserting an additional copy of said genes and orf's into the chromosome of a suitable strain using the method of Hamilton et al (Journal of Bacteriology 171: 4617-4622 (1989)).

By measures of over expression the activity or concentration of the polypeptide or enzyme is in general increased by at least 10%, 25%, 50%, 75%, 100%, 150%, 200%, 300%, 400% or 500%, up to a maximum of 1000% or 2000%, compared to the activity or concentration of unmodified strain.

Measures of decreasing expression include using weak promoters like those described by Kawano et al. (Nucleic Acids Research 33(19), 6268-6276 (2005)) or removing the promoter sequence while maintaining the ribosome binding site. Decreased expression can also be achieved by introducing stop mutations into the coding sequence of said orf's and suppressing the stop mutations by appropriate t-RNA suppressors. This method is described in WO 03/074719.

Measures of eliminating expression include deletion or insertion of at least one nucleobase within the coding sequence of said genes and orf's.

By measures of decreasing or eliminating expression the activity or concentration of the polypeptide or enzyme is in general reduced to 0 to 75%, 0 to 50%, 0 to 25%, 0 to 10% or 0 to 5% of the activity or concentration in the unmodified strain.

In particular it was found that overexpression of the yohJ orf (see f)) in a strain like MG442 was beneficial for L-threonine formation while decreasing of expression in a strain like KCCM-10132 improved production parameters. Similar results were obtained using the genetic elements described under a), b), c), e), and f). Evidently these genes and orf's are targets for the improvement of L-amino acid producers, in particular L-threonine and L-homoserine producers.

Improvement means an increase of at least 0,5 % of a parameter selected from the group consisting of concentration (g/l), yield (g amino acid produced/g carbon source consumed) and product formation rate (g/l*h) as compared to the performance of the unmodified strain in a fermentation process.

The strains having the characteristics described above can be used in batch, fed batch, repeated fed batch and continuous cultures. The amino acids are collected from the broth and purified. Likewise products can be manufactured, which contain residues of the fermentation broth.

The measures described herewith also apply for improvement of processes for the production of other L-amino acids in particular L-isoleucine, L-lysine and L-valine.

## Claims

1. A process for the production of an L-amino acid comprising the following steps
a) modulating in the genus Escherichia the expression of of one or more of the orf's and genes selected from the group consisting of nrdH, nrdI, nrdE, nrdF, ybaL, ygaC, yohJ, yjgP and yggW,
b) culturing said modified strains in a suitable culture medium, and
c) collecting said L-amino acids.

2. The process of claim 1, wherein said L-amino acid is L-threonine or L-homoserine.

3. The process of claim 1, wherein said Escherichia is Escherichia coli.


SEQUENCE LISTING

<110>  Degussa AG

<120>  Methods for the Production of L-Threonine

<130>  2006E00133

<160>  22

<170>  PatentIn version 3.3

<210>  1
<211>  1677
<212>  DNA
<213>  Escherichia coli K12


<220>
<221>  CDS
<222>  (1)..(1674)
<223>  ybaL

<400>  1
atg cat cac gcc acc ccg ctt atc acc acc att gtt ggc ggc ctt gtg      48
Met His His Ala Thr Pro Leu Ile Thr Thr Ile Val Gly Gly Leu Val
1               5                   10                  15

ctc gcc ttt atc ctc ggc atg ctg gcc aat aaa cta cgt att tct cct      96
Leu Ala Phe Ile Leu Gly Met Leu Ala Asn Lys Leu Arg Ile Ser Pro
            20                  25                  30

ctg gtg gga tat ctg tta gcg ggt gtg ctg gca gga cca ttc act ccg      144
Leu Val Gly Tyr Leu Leu Ala Gly Val Leu Ala Gly Pro Phe Thr Pro
        35                  40                  45

ggc ttt gtt gcc gat acc aag ctt gcc ccg gaa ctg gct gaa ctg ggc      192
Gly Phe Val Ala Asp Thr Lys Leu Ala Pro Glu Leu Ala Glu Leu Gly
    50                  55                  60

gtc att ctg ttg atg ttt ggc gtc ggt ttg cac ttt tcg ctg aag gat      240
Val Ile Leu Leu Met Phe Gly Val Gly Leu His Phe Ser Leu Lys Asp
65                  70                  75                  80

ttg atg gcg gta aag gcc atc gcc att ccc ggt gcg atc gcc cag ata      288
Leu Met Ala Val Lys Ala Ile Ala Ile Pro Gly Ala Ile Ala Gln Ile
                85                  90                  95

gcc gtg gcg acg ctg ctg ggt atg gcg ctc tct gcc gtg ctg ggc tgg      336
Ala Val Ala Thr Leu Leu Gly Met Ala Leu Ser Ala Val Leu Gly Trp
            100                 105                 110

tcg tta atg acc ggt atc gtg ttc ggt tta tgt ctt tcc acc gcc agt      384
Ser Leu Met Thr Gly Ile Val Phe Gly Leu Cys Leu Ser Thr Ala Ser
        115                 120                 125

acc gtg gtg tta ctg cgc gca ctt gaa gaa cgg caa tta att gac agt      432
Thr Val Val Leu Leu Arg Ala Leu Glu Glu Arg Gln Leu Ile Asp Ser
    130                 135                 140

cag cgt ggg caa atc gcc atc ggt tgg ttg att gtg gaa gac ctg gta      480
Gln Arg Gly Gln Ile Ala Ile Gly Trp Leu Ile Val Glu Asp Leu Val
145                 150                 155                 160

atg gtt ctg acg ctg gtg ttg ctg ccc gca gtg gca gga atg atg gaa      528

Met Val Leu Thr Leu Val Leu Leu Pro Ala Val Ala Gly Met Met Glu
                165                 170                 175

cag ggc gat gtg ggc ttt gcc act ctt gca gtc gat atg ggg atc acc      576
Gln Gly Asp Val Gly Phe Ala Thr Leu Ala Val Asp Met Gly Ile Thr
            180                 185                 190

atc ggc aaa gtg atc gca ttt atc gcc att atg atg ctg gta ggt cgc      624
Ile Gly Lys Val Ile Ala Phe Ile Ala Ile Met Met Leu Val Gly Arg
        195                 200                 205

cgt ctg gtg ccg tgg att atg gca cgc agc gcg gca acc ggt tct cgc      672
Arg Leu Val Pro Trp Ile Met Ala Arg Ser Ala Ala Thr Gly Ser Arg
    210                 215                 220

gag ctg ttt acc ctg tcg gtg ctg gcg ctg gcg tta ggg gtt gcc ttt      720
Glu Leu Phe Thr Leu Ser Val Leu Ala Leu Ala Leu Gly Val Ala Phe
225                 230                 235                 240

ggt gcg gta gag ctg ttt gat gtc tcc ttt gca ctc ggt gcg ttc ttt      768
Gly Ala Val Glu Leu Phe Asp Val Ser Phe Ala Leu Gly Ala Phe Phe
                245                 250                 255

gcc ggg atg gta ctg aac gag tct gaa ctg agt cac cgt gcc gcc cac      816
Ala Gly Met Val Leu Asn Glu Ser Glu Leu Ser His Arg Ala Ala His
            260                 265                 270

gat acg ctg cca ttg cgc gac gcg ttt gcg gtg ctg ttt ttt gtc tcc      864
Asp Thr Leu Pro Leu Arg Asp Ala Phe Ala Val Leu Phe Phe Val Ser
        275                 280                 285

gtc ggg atg ttg ttt gat ccg tta att ctg att cag caa ccg ctg gca      912
Val Gly Met Leu Phe Asp Pro Leu Ile Leu Ile Gln Gln Pro Leu Ala
    290                 295                 300

gtg ctg gcg acg ctg gcg att att ctg ttt ggt aag tcg tta gcc gca      960
Val Leu Ala Thr Leu Ala Ile Ile Leu Phe Gly Lys Ser Leu Ala Ala
305                 310                 315                 320

ttt ttc ctg gtg cga ctg ttt ggt cac tcc caa cgt acg gca tta acc     1008
Phe Phe Leu Val Arg Leu Phe Gly His Ser Gln Arg Thr Ala Leu Thr
                325                 330                 335

atc gcc gcc agc ctg gcg cag att ggt gag ttc gcg ttt atc ctg gcg     1056
Ile Ala Ala Ser Leu Ala Gln Ile Gly Glu Phe Ala Phe Ile Leu Ala
            340                 345                 350

gga ctg gga atg gca ttg aat tta ctg ccg cag gcc gga caa aac ctg     1104
Gly Leu Gly Met Ala Leu Asn Leu Leu Pro Gln Ala Gly Gln Asn Leu
        355                 360                 365

gta ctg gca ggg gcg atc ctg tcg att atg ctc aac ccg gta ctg ttc     1152
Val Leu Ala Gly Ala Ile Leu Ser Ile Met Leu Asn Pro Val Leu Phe
    370                 375                 380

gca cta ctg gag aaa tat ctg gcg aag acc gaa acg ctg gaa gag cag     1200
Ala Leu Leu Glu Lys Tyr Leu Ala Lys Thr Glu Thr Leu Glu Glu Gln
385                 390                 395                 400

acg ctg gaa gag gca atc gaa gaa gag aag cag atc cca gtg gat att     1248
Thr Leu Glu Glu Ala Ile Glu Glu Glu Lys Gln Ile Pro Val Asp Ile
                405                 410                 415

tgc aac cat gcg cta ctg gtg ggt tac ggt cgt gta ggc agc ctg ctg     1296
Cys Asn His Ala Leu Leu Val Gly Tyr Gly Arg Val Gly Ser Leu Leu

420                 425                 430

ggg gag aaa ttg ctc gcc tct gat att ccg ctg gtg gtg att gag acg      1344
Gly Glu Lys Leu Leu Ala Ser Asp Ile Pro Leu Val Val Ile Glu Thr
        435                 440                 445

tca cga acc cgt gtt gat gag ctg cga gag cgc ggg gtc cgc gca gta      1392
Ser Arg Thr Arg Val Asp Glu Leu Arg Glu Arg Gly Val Arg Ala Val
    450                 455                 460

ttg ggc aat gcg gcg aac gaa gaa att atg caa ctg gcg cat ctg gaa      1440
Leu Gly Asn Ala Ala Asn Glu Glu Ile Met Gln Leu Ala His Leu Glu
465                 470                 475                 480

tgt gca aaa tgg ctg atc ctg acg att ccc aac ggt tat gaa gcg ggt      1488
Cys Ala Lys Trp Leu Ile Leu Thr Ile Pro Asn Gly Tyr Glu Ala Gly
                485                 490                 495

gag att gtg gca tct gcc cgc gcg aaa aat ccg gat att gag att att      1536
Glu Ile Val Ala Ser Ala Arg Ala Lys Asn Pro Asp Ile Glu Ile Ile
            500                 505                 510

gcc cgc gcc cat tat gac gat gaa gtg gcg tat atc acc gaa cgt ggt      1584
Ala Arg Ala His Tyr Asp Asp Glu Val Ala Tyr Ile Thr Glu Arg Gly
        515                 520                 525

gcg aat cag gta gtg atg ggc gag cgt gaa atc gcc cgt acc atg ctg      1632
Ala Asn Gln Val Val Met Gly Glu Arg Glu Ile Ala Arg Thr Met Leu
    530                 535                 540

gaa ctg ctg gaa acg cca ccg gcg ggt gag gtg gtg acg ggg taa          1677
Glu Leu Leu Glu Thr Pro Pro Ala Gly Glu Val Val Thr Gly
545                 550                 555


<210>  2
<211>  558
<212>  PRT
<213>  Escherichia coli K12

<400>  2

Met His His Ala Thr Pro Leu Ile Thr Thr Ile Val Gly Gly Leu Val
1               5                   10                  15


Leu Ala Phe Ile Leu Gly Met Leu Ala Asn Lys Leu Arg Ile Ser Pro
            20                  25                  30


Leu Val Gly Tyr Leu Leu Ala Gly Val Leu Ala Gly Pro Phe Thr Pro
        35                  40                  45


Gly Phe Val Ala Asp Thr Lys Leu Ala Pro Glu Leu Ala Glu Leu Gly
    50                  55                  60


Val Ile Leu Leu Met Phe Gly Val Gly Leu His Phe Ser Leu Lys Asp
65                  70                  75                  80


Leu Met Ala Val Lys Ala Ile Ala Ile Pro Gly Ala Ile Ala Gln Ile
                85                  90                  95

Ala Val Ala Thr Leu Leu Gly Met Ala Leu Ser Ala Val Leu Gly Trp
            100                 105                 110

Ser Leu Met Thr Gly Ile Val Phe Gly Leu Cys Leu Ser Thr Ala Ser
        115                 120                 125

Thr Val Val Leu Leu Arg Ala Leu Glu Glu Arg Gln Leu Ile Asp Ser
    130                 135                 140

Gln Arg Gly Gln Ile Ala Ile Gly Trp Leu Ile Val Glu Asp Leu Val
145                 150                 155                 160

Met Val Leu Thr Leu Val Leu Leu Pro Ala Val Ala Gly Met Met Glu
                165                 170                 175

Gln Gly Asp Val Gly Phe Ala Thr Leu Ala Val Asp Met Gly Ile Thr
            180                 185                 190

Ile Gly Lys Val Ile Ala Phe Ile Ala Ile Met Met Leu Val Gly Arg
        195                 200                 205

Arg Leu Val Pro Trp Ile Met Ala Arg Ser Ala Ala Thr Gly Ser Arg
    210                 215                 220

Glu Leu Phe Thr Leu Ser Val Leu Ala Leu Ala Leu Gly Val Ala Phe
225                 230                 235                 240

Gly Ala Val Glu Leu Phe Asp Val Ser Phe Ala Leu Gly Ala Phe Phe
                245                 250                 255

Ala Gly Met Val Leu Asn Glu Ser Glu Leu Ser His Arg Ala Ala His
            260                 265                 270

Asp Thr Leu Pro Leu Arg Asp Ala Phe Ala Val Leu Phe Phe Val Ser
        275                 280                 285

Val Gly Met Leu Phe Asp Pro Leu Ile Leu Ile Gln Gln Pro Leu Ala
    290                 295                 300

Val Leu Ala Thr Leu Ala Ile Ile Leu Phe Gly Lys Ser Leu Ala Ala
305                 310                 315                 320

Phe Phe Leu Val Arg Leu Phe Gly His Ser Gln Arg Thr Ala Leu Thr
                325                 330                 335

Ile Ala Ala Ser Leu Ala Gln Ile Gly Glu Phe Ala Phe Ile Leu Ala
            340                 345                 350

Gly Leu Gly Met Ala Leu Asn Leu Leu Pro Gln Ala Gly Gln Asn Leu
        355                 360                 365

Val Leu Ala Gly Ala Ile Leu Ser Ile Met Leu Asn Pro Val Leu Phe
    370                 375                 380

Ala Leu Leu Glu Lys Tyr Leu Ala Lys Thr Glu Thr Leu Glu Glu Gln
385                 390                 395                 400

Thr Leu Glu Glu Ala Ile Glu Glu Glu Lys Gln Ile Pro Val Asp Ile
                405                 410                 415

Cys Asn His Ala Leu Leu Val Gly Tyr Gly Arg Val Gly Ser Leu Leu
            420                 425                 430

Gly Glu Lys Leu Leu Ala Ser Asp Ile Pro Leu Val Val Ile Glu Thr
        435                 440                 445

Ser Arg Thr Arg Val Asp Glu Leu Arg Glu Arg Gly Val Arg Ala Val
    450                 455                 460

Leu Gly Asn Ala Ala Asn Glu Glu Ile Met Gln Leu Ala His Leu Glu
465                 470                 475                 480

Cys Ala Lys Trp Leu Ile Leu Thr Ile Pro Asn Gly Tyr Glu Ala Gly
                485                 490                 495

Glu Ile Val Ala Ser Ala Arg Ala Lys Asn Pro Asp Ile Glu Ile Ile
            500                 505                 510

Ala Arg Ala His Tyr Asp Asp Glu Val Ala Tyr Ile Thr Glu Arg Gly
        515                 520                 525

Ala Asn Gln Val Val Met Gly Glu Arg Glu Ile Ala Arg Thr Met Leu
    530                 535                 540

Glu Leu Leu Glu Thr Pro Pro Ala Gly Glu Val Val Thr Gly
545                 550                 555

<210>  3
<211>  1677
<212>  DNA
<213>  Escherichia coli O157:H7

<220>
<221>  CDS
<222>  (1)..(1674)
<223>  ybaL

<400>  3
atg cat cac gcc acc ccg ctt atc acc acc att gtt ggc ggc ctt gtg        48

Met His His Ala Thr Pro Leu Ile Thr Thr Ile Val Gly Gly Leu Val
1               5                   10                  15

ctc gcc ttt atc ctc ggc atg ctg gcc aat aaa cta cgt att tct cct      96
Leu Ala Phe Ile Leu Gly Met Leu Ala Asn Lys Leu Arg Ile Ser Pro
            20                  25                  30

ctg gtg gga tat ctg tta gcg ggt gtg ctg gca gga cca ttc act ccg      144
Leu Val Gly Tyr Leu Leu Ala Gly Val Leu Ala Gly Pro Phe Thr Pro
        35                  40                  45

ggc ttt gtt gcc gat acc aag ctt gcg ccg gaa ctg gct gaa ctg ggc      192
Gly Phe Val Ala Asp Thr Lys Leu Ala Pro Glu Leu Ala Glu Leu Gly
    50                  55                  60

gtc att ctg ttg atg ttt ggc gtg ggt ctg cac ttt tcg ctc aag gat      240
Val Ile Leu Leu Met Phe Gly Val Gly Leu His Phe Ser Leu Lys Asp
65                  70                  75                  80

ttg atg gcg gta aag gcc atc gcc att ccc ggt gcg atc gcc cag ata      288
Leu Met Ala Val Lys Ala Ile Ala Ile Pro Gly Ala Ile Ala Gln Ile
                85                  90                  95

gcc gtg gcg acg ctg ctg ggt atg gcg ctc tct gcc gtg ctg ggc tgg      336
Ala Val Ala Thr Leu Leu Gly Met Ala Leu Ser Ala Val Leu Gly Trp
            100                 105                 110

tcg tta atg acc ggt atc gtg ttc ggt tta tgt ctt tcc acc gcc agt      384
Ser Leu Met Thr Gly Ile Val Phe Gly Leu Cys Leu Ser Thr Ala Ser
        115                 120                 125

acc gtg gtg tta ctg cgc gca ctt gaa gaa cgg caa tta att gac agt      432
Thr Val Val Leu Leu Arg Ala Leu Glu Glu Arg Gln Leu Ile Asp Ser
    130                 135                 140

cag cgt ggg caa atc gcc atc ggt tgg ttg att gtg gaa gac ctg gta      480
Gln Arg Gly Gln Ile Ala Ile Gly Trp Leu Ile Val Glu Asp Leu Val
145                 150                 155                 160

atg gtt ctg acg ctg gtg ttg ctg ccc gca gtg gca gga atg atg gaa      528
Met Val Leu Thr Leu Val Leu Leu Pro Ala Val Ala Gly Met Met Glu
                165                 170                 175

cag ggc gat gtg ggc ttt gcc act ctt gca gtc gat atg ggg atc acc      576
Gln Gly Asp Val Gly Phe Ala Thr Leu Ala Val Asp Met Gly Ile Thr
            180                 185                 190

atc ggc aaa gtg atc gcg ttt atc gcc att atg atg ctg gta ggt cgc      624
Ile Gly Lys Val Ile Ala Phe Ile Ala Ile Met Met Leu Val Gly Arg
        195                 200                 205

cgt ctg gtg ccg tgg att atg gca cgc agc gcg gca acc ggt tct cgc      672
Arg Leu Val Pro Trp Ile Met Ala Arg Ser Ala Ala Thr Gly Ser Arg
    210                 215                 220

gag ctg ttt acc ctg tcg gtg ctg gcg ctg gcg tta ggg att gcc ttt      720
Glu Leu Phe Thr Leu Ser Val Leu Ala Leu Ala Leu Gly Ile Ala Phe
225                 230                 235                 240

ggt gcg gta gag ctg ttt gat gtc tcc ttt gca ctc ggt gcg ttc ttt      768
Gly Ala Val Glu Leu Phe Asp Val Ser Phe Ala Leu Gly Ala Phe Phe
                245                 250                 255

gcc ggg atg gta ctg aac gag tct gaa ctg agt cac cgt gcc gcc cac      816
Ala Gly Met Val Leu Asn Glu Ser Glu Leu Ser His Arg Ala Ala His

            260                 265                 270

gat acg ctg cca ttg cgc gac gcg ttt gcg gtg ctg ttt ttt gtc tcc 864
Asp Thr Leu Pro Leu Arg Asp Ala Phe Ala Val Leu Phe Phe Val Ser
        275                 280                 285

gtc ggg atg ttg ttt gat ccg tta att ctg att cag caa ccg ctg gca 912
Val Gly Met Leu Phe Asp Pro Leu Ile Leu Ile Gln Gln Pro Leu Ala
    290                 295                 300

gtg ctg gcg acg ctg gcg att att ctg ttt ggt aag tcg tta gcc gcg 960
Val Leu Ala Thr Leu Ala Ile Ile Leu Phe Gly Lys Ser Leu Ala Ala
305                 310                 315                 320

ttt ttc ctg gtg cga ctg ttt ggt cac tcc caa cgt acg gca tta act 1008
Phe Phe Leu Val Arg Leu Phe Gly His Ser Gln Arg Thr Ala Leu Thr
                325                 330                 335

atc gcc gcc agc ctg gcg cag att ggt gag ttc gcg ttt atc ctg gcg 1056
Ile Ala Ala Ser Leu Ala Gln Ile Gly Glu Phe Ala Phe Ile Leu Ala
            340                 345                 350

gga ctg gga atg gca ttg aat tta ctg ccg cag gcc gga caa aac ctg 1104
Gly Leu Gly Met Ala Leu Asn Leu Leu Pro Gln Ala Gly Gln Asn Leu
        355                 360                 365

gta ctg gca ggg gcg atc ctg tcg att atg ctc aac cca gta ctg ttc 1152
Val Leu Ala Gly Ala Ile Leu Ser Ile Met Leu Asn Pro Val Leu Phe
    370                 375                 380

gca cta ctg gag aaa tat ctg gcg aag acc gaa acg ctg gaa gag cag 1200
Ala Leu Leu Glu Lys Tyr Leu Ala Lys Thr Glu Thr Leu Glu Glu Gln
385                 390                 395                 400

acg ctg gaa gag gca atc gaa gaa gag aag cag atc cca gtg gat att 1248
Thr Leu Glu Glu Ala Ile Glu Glu Glu Lys Gln Ile Pro Val Asp Ile
                405                 410                 415

tgc aac cat gcg cta ttg gtg ggt tac ggt cgt gta ggc agc ctg ctg 1296
Cys Asn His Ala Leu Leu Val Gly Tyr Gly Arg Val Gly Ser Leu Leu
            420                 425                 430

ggg gag aaa ttg ctc gcc tct gat att ccg ctg gtg gtg att gag acg 1344
Gly Glu Lys Leu Leu Ala Ser Asp Ile Pro Leu Val Val Ile Glu Thr
        435                 440                 445

tca cga acc cgt gtt gat gag ctg cga gag cgc ggg gtc cgc gca gta 1392
Ser Arg Thr Arg Val Asp Glu Leu Arg Glu Arg Gly Val Arg Ala Val
    450                 455                 460

ttg ggc aat gcg gcg aac gaa gaa att atg caa ctg gcg cat ctg gaa 1440
Leu Gly Asn Ala Ala Asn Glu Glu Ile Met Gln Leu Ala His Leu Glu
465                 470                 475                 480

tgt gca aaa tgg ctg atc ctg acg atc ccc aac ggt tat gaa gcg ggt 1488
Cys Ala Lys Trp Leu Ile Leu Thr Ile Pro Asn Gly Tyr Glu Ala Gly
                485                 490                 495

gag att gtg gca tct gcc cgc gcg aaa aat ccg gat att gag att att 1536
Glu Ile Val Ala Ser Ala Arg Ala Lys Asn Pro Asp Ile Glu Ile Ile
            500                 505                 510

gcc cgc gcc cat tat gac gat gaa gtg gcg tat atc acc gaa cgt ggt 1584
Ala Arg Ala His Tyr Asp Asp Glu Val Ala Tyr Ile Thr Glu Arg Gly
        515                 520                 525

gcg aat caa gta gtg atg ggc gag cgt gaa att gcc cgt act atg ctg      1632
Ala Asn Gln Val Val Met Gly Glu Arg Glu Ile Ala Arg Thr Met Leu
    530                 535                 540

gaa ctg ctg gaa acg cca ccg gcg ggt gaa gtg gtg acg ggg taa         1677
Glu Leu Leu Glu Thr Pro Pro Ala Gly Glu Val Val Thr Gly
545                 550                 555

<210>  4
<211>  558
<212>  PRT
<213>  Escherichia coli O157:H7

<400>  4

Met His His Ala Thr Pro Leu Ile Thr Thr Ile Val Gly Gly Leu Val
1               5                   10                  15

Leu Ala Phe Ile Leu Gly Met Leu Ala Asn Lys Leu Arg Ile Ser Pro
            20                  25                  30

Leu Val Gly Tyr Leu Leu Ala Gly Val Leu Ala Gly Pro Phe Thr Pro
        35                  40                  45

Gly Phe Val Ala Asp Thr Lys Leu Ala Pro Glu Leu Ala Glu Leu Gly
    50                  55                  60

Val Ile Leu Leu Met Phe Gly Val Gly Leu His Phe Ser Leu Lys Asp
65                  70                  75                  80

Leu Met Ala Val Lys Ala Ile Ala Ile Pro Gly Ala Ile Ala Gln Ile
                85                  90                  95

Ala Val Ala Thr Leu Leu Gly Met Ala Leu Ser Ala Val Leu Gly Trp
            100                 105                 110

Ser Leu Met Thr Gly Ile Val Phe Gly Leu Cys Leu Ser Thr Ala Ser
        115                 120                 125

Thr Val Val Leu Leu Arg Ala Leu Glu Glu Arg Gln Leu Ile Asp Ser
    130                 135                 140

Gln Arg Gly Gln Ile Ala Ile Gly Trp Leu Ile Val Glu Asp Leu Val
145                 150                 155                 160

Met Val Leu Thr Leu Val Leu Leu Pro Ala Val Ala Gly Met Met Glu
                165                 170                 175

Gln Gly Asp Val Gly Phe Ala Thr Leu Ala Val Asp Met Gly Ile Thr
            180                 185                 190

Ile Gly Lys Val Ile Ala Phe Ile Ala Ile Met Met Leu Val Gly Arg
        195                 200                 205

Arg Leu Val Pro Trp Ile Met Ala Arg Ser Ala Ala Thr Gly Ser Arg
    210                 215                 220

Glu Leu Phe Thr Leu Ser Val Leu Ala Leu Ala Leu Gly Ile Ala Phe
225                 230                 235                 240

Gly Ala Val Glu Leu Phe Asp Val Ser Phe Ala Leu Gly Ala Phe Phe
                245                 250                 255

Ala Gly Met Val Leu Asn Glu Ser Glu Leu Ser His Arg Ala Ala His
            260                 265                 270

Asp Thr Leu Pro Leu Arg Asp Ala Phe Ala Val Leu Phe Phe Val Ser
        275                 280                 285

Val Gly Met Leu Phe Asp Pro Leu Ile Leu Ile Gln Gln Pro Leu Ala
    290                 295                 300

Val Leu Ala Thr Leu Ala Ile Ile Leu Phe Gly Lys Ser Leu Ala Ala
305                 310                 315                 320

Phe Phe Leu Val Arg Leu Phe Gly His Ser Gln Arg Thr Ala Leu Thr
                325                 330                 335

Ile Ala Ala Ser Leu Ala Gln Ile Gly Glu Phe Ala Phe Ile Leu Ala
            340                 345                 350

Gly Leu Gly Met Ala Leu Asn Leu Leu Pro Gln Ala Gly Gln Asn Leu
        355                 360                 365

Val Leu Ala Gly Ala Ile Leu Ser Ile Met Leu Asn Pro Val Leu Phe
    370                 375                 380

Ala Leu Leu Glu Lys Tyr Leu Ala Lys Thr Glu Thr Leu Glu Glu Gln
385                 390                 395                 400

Thr Leu Glu Glu Ala Ile Glu Glu Glu Lys Gln Ile Pro Val Asp Ile
                405                 410                 415

Cys Asn His Ala Leu Leu Val Gly Tyr Gly Arg Val Gly Ser Leu Leu
            420                 425                 430

Gly Glu Lys Leu Leu Ala Ser Asp Ile Pro Leu Val Val Ile Glu Thr
        435                 440                 445

Ser Arg Thr Arg Val Asp Glu Leu Arg Glu Arg Gly Val Arg Ala Val

450                 455                 460

Leu Gly Asn Ala Ala Asn Glu Glu Ile Met Gln Leu Ala His Leu Glu
465                 470                 475                 480

Cys Ala Lys Trp Leu Ile Leu Thr Ile Pro Asn Gly Tyr Glu Ala Gly
                485                 490                 495

Glu Ile Val Ala Ser Ala Arg Ala Lys Asn Pro Asp Ile Glu Ile Ile
            500                 505                 510

Ala Arg Ala His Tyr Asp Asp Glu Val Ala Tyr Ile Thr Glu Arg Gly
        515                 520                 525

Ala Asn Gln Val Val Met Gly Glu Arg Glu Ile Ala Arg Thr Met Leu
    530                 535                 540

Glu Leu Leu Glu Thr Pro Pro Ala Gly Glu Val Val Thr Gly
545                 550                 555

<210>  5
<211>  1677
<212>  DNA
<213>  Escherichia coli CFT073

<220>
<221>  CDS
<222>  (1)..(1674)
<223>  ybaL

<400>  5
atg cat cac gcc acc ccg ctt atc acc acc att gtt ggc ggc ctt gtg       48
Met His His Ala Thr Pro Leu Ile Thr Thr Ile Val Gly Gly Leu Val
1               5                   10                  15

ctc gcc ttt atc ctc ggc atg ctg gcc aat aaa cta cgt att tct cct       96
Leu Ala Phe Ile Leu Gly Met Leu Ala Asn Lys Leu Arg Ile Ser Pro
            20                  25                  30

ctg gtg gga tat ctg tta gcg ggt gtg ctg gca gga cca ttc act ccg      144
Leu Val Gly Tyr Leu Leu Ala Gly Val Leu Ala Gly Pro Phe Thr Pro
        35                  40                  45

ggc ttt gtt gcc gat acc aag ctt gcg ccg gaa ctg gct gaa ctg ggc      192
Gly Phe Val Ala Asp Thr Lys Leu Ala Pro Glu Leu Ala Glu Leu Gly
    50                  55                  60

gtt att ttg ctg atg ttc ggc gtc ggt ttg cat ttt tcg ctc aag gat      240
Val Ile Leu Leu Met Phe Gly Val Gly Leu His Phe Ser Leu Lys Asp
65                  70                  75                  80

ttg atg gcg gta aag tcc atc gcc att ccc ggc gcg atc gcc cag ata      288
Leu Met Ala Val Lys Ser Ile Ala Ile Pro Gly Ala Ile Ala Gln Ile
                85                  90                  95

gcc gtg gcg acg ctg ctg ggt atg gcg ctc tcc gcc gtg ctg ggc tgg      336
Ala Val Ala Thr Leu Leu Gly Met Ala Leu Ser Ala Val Leu Gly Trp

            100                 105                 110

tcg tta atg acc ggt atc gtg ttc ggc tta tgt ctt tcc acc gcc agt 384
Ser Leu Met Thr Gly Ile Val Phe Gly Leu Cys Leu Ser Thr Ala Ser
        115                 120                 125

acc gtg gtg tta ctg cgc gca ctt gaa gaa cgg caa tta att gac agt 432
Thr Val Val Leu Leu Arg Ala Leu Glu Glu Arg Gln Leu Ile Asp Ser
    130                 135                 140

cag cgt ggg caa atc gcc atc ggt tgg ttg att gtg gaa gac ctg gtg 480
Gln Arg Gly Gln Ile Ala Ile Gly Trp Leu Ile Val Glu Asp Leu Val
145                 150                 155                 160

atg gtc ctg acg ctg gta ttg cta ccc gcg gta gca gga atg atg gaa 528
Met Val Leu Thr Leu Val Leu Leu Pro Ala Val Ala Gly Met Met Glu
                165                 170                 175

cag ggc gat gtg ggc ttt gcc act ctt gca gtc gat atg ggg atc acc 576
Gln Gly Asp Val Gly Phe Ala Thr Leu Ala Val Asp Met Gly Ile Thr
            180                 185                 190

atc ggc aag gtg atc gca ttt atc gcc att atg atg ttg gta ggt cgc 624
Ile Gly Lys Val Ile Ala Phe Ile Ala Ile Met Met Leu Val Gly Arg
        195                 200                 205

cgt ctg gtg ccg tgg att atg gca cgc agc gca gca acc ggt tcc cgc 672
Arg Leu Val Pro Trp Ile Met Ala Arg Ser Ala Ala Thr Gly Ser Arg
    210                 215                 220

gag ctg ttt acc ctg tcg gtg cta gcg ctg gcg tta ggg att gcc ttt 720
Glu Leu Phe Thr Leu Ser Val Leu Ala Leu Ala Leu Gly Ile Ala Phe
225                 230                 235                 240

ggt gcg gta gag ctg ttt gat gtc tcc ttt gca ctc ggt gcg ttc ttt 768
Gly Ala Val Glu Leu Phe Asp Val Ser Phe Ala Leu Gly Ala Phe Phe
                245                 250                 255

gcc ggg atg gta ctg aac gag tct gaa ctg agt cac cgt gcc gcc cac 816
Ala Gly Met Val Leu Asn Glu Ser Glu Leu Ser His Arg Ala Ala His
            260                 265                 270

gat aca ctg cca ttg cgc gac gcg ttt gcg gtg ctg ttt ttt gtc tcc 864
Asp Thr Leu Pro Leu Arg Asp Ala Phe Ala Val Leu Phe Phe Val Ser
        275                 280                 285

gtc ggg atg ttg ttt gat ccg tta att ctg att cag caa ccg ctg gca 912
Val Gly Met Leu Phe Asp Pro Leu Ile Leu Ile Gln Gln Pro Leu Ala
    290                 295                 300

gta ctg gcg acg ctg gca att att ctg ttt ggt aaa tcg gtc gcg gcc 960
Val Leu Ala Thr Leu Ala Ile Ile Leu Phe Gly Lys Ser Val Ala Ala
305                 310                 315                 320

ctg ttc ctt gtg cgt ttg ttt ggt cac tcc cag cgc acg gcg tta acc 1008
Leu Phe Leu Val Arg Leu Phe Gly His Ser Gln Arg Thr Ala Leu Thr
                325                 330                 335

atc gcc gcc agc ctg gcg cag att ggt gag ttc gcg ttt atc ctg gcg 1056
Ile Ala Ala Ser Leu Ala Gln Ile Gly Glu Phe Ala Phe Ile Leu Ala
            340                 345                 350

gga cta gga atg gca ttg aat tta ctg ccg cag gcc gga caa aac ctg 1104
Gly Leu Gly Met Ala Leu Asn Leu Leu Pro Gln Ala Gly Gln Asn Leu
        355                 360                 365

gta ctg gca ggg gcg atc ctg tcg att atg ctc aac ccg gta ctg ttc     1152
Val Leu Ala Gly Ala Ile Leu Ser Ile Met Leu Asn Pro Val Leu Phe
    370                 375                 380

gca cta ctg gaa aaa tat ctg gcg aag acc gaa acg ctg gaa gag cag     1200
Ala Leu Leu Glu Lys Tyr Leu Ala Lys Thr Glu Thr Leu Glu Glu Gln
385                 390                 395                 400

acg ctg gaa gag gca atc gaa gaa gag aag cag atc cca gtg gat att     1248
Thr Leu Glu Glu Ala Ile Glu Glu Glu Lys Gln Ile Pro Val Asp Ile
                405                 410                 415

tgc aac cat gcg cta ctg gtg ggt tac ggt cgt gta ggc agc ctg ctg     1296
Cys Asn His Ala Leu Leu Val Gly Tyr Gly Arg Val Gly Ser Leu Leu
            420                 425                 430

ggg gag aaa ttg ctc gcc tct gat att ccg ctg gtg gtg att gag acg     1344
Gly Glu Lys Leu Leu Ala Ser Asp Ile Pro Leu Val Val Ile Glu Thr
        435                 440                 445

tca cga act cgt gtt gat gag ctg cgc gag cgc ggg gtc cgc gca gta     1392
Ser Arg Thr Arg Val Asp Glu Leu Arg Glu Arg Gly Val Arg Ala Val
    450                 455                 460

ttg ggc aat gcg gcg aac gaa gaa att atg caa ctg gcg cat ctg gaa     1440
Leu Gly Asn Ala Ala Asn Glu Glu Ile Met Gln Leu Ala His Leu Glu
465                 470                 475                 480

tgt gca aaa tgg ctg atc ctg acg att ccc aac ggc tat gaa gcg ggc     1488
Cys Ala Lys Trp Leu Ile Leu Thr Ile Pro Asn Gly Tyr Glu Ala Gly
                485                 490                 495

gag att gtg gca tct gcc cgc gcg aaa aat ccg gat att gag att att     1536
Glu Ile Val Ala Ser Ala Arg Ala Lys Asn Pro Asp Ile Glu Ile Ile
            500                 505                 510

gcc cgc gcc cat tat gac gat gaa gtg gcg tat atc acc gaa cgt ggt     1584
Ala Arg Ala His Tyr Asp Asp Glu Val Ala Tyr Ile Thr Glu Arg Gly
        515                 520                 525

gcg aat cag gta gtg atg ggt gag cgt gaa att gcc cgt act atg ctg     1632
Ala Asn Gln Val Val Met Gly Glu Arg Glu Ile Ala Arg Thr Met Leu
    530                 535                 540

gaa ctg ctg gaa acg cca ccg gcg ggt gag gtg gtg acg agg taa         1677
Glu Leu Leu Glu Thr Pro Pro Ala Gly Glu Val Val Thr Arg
545                 550                 555

<210>  6
<211>  558
<212>  PRT
<213>  Escherichia coli CFT073

<400>  6

Met His His Ala Thr Pro Leu Ile Thr Thr Ile Val Gly Gly Leu Val
1               5                   10                  15

Leu Ala Phe Ile Leu Gly Met Leu Ala Asn Lys Leu Arg Ile Ser Pro
            20                  25                  30

Leu Val Gly Tyr Leu Leu Ala Gly Val Leu Ala Gly Pro Phe Thr Pro
        35                  40                  45

Gly Phe Val Ala Asp Thr Lys Leu Ala Pro Glu Leu Ala Glu Leu Gly
    50                  55                  60

Val Ile Leu Leu Met Phe Gly Val Gly Leu His Phe Ser Leu Lys Asp
65                  70                  75                  80

Leu Met Ala Val Lys Ser Ile Ala Ile Pro Gly Ala Ile Ala Gln Ile
                85                  90                  95

Ala Val Ala Thr Leu Leu Gly Met Ala Leu Ser Ala Val Leu Gly Trp
            100                 105                 110

Ser Leu Met Thr Gly Ile Val Phe Gly Leu Cys Leu Ser Thr Ala Ser
        115                 120                 125

Thr Val Val Leu Leu Arg Ala Leu Glu Glu Arg Gln Leu Ile Asp Ser
    130                 135                 140

Gln Arg Gly Gln Ile Ala Ile Gly Trp Leu Ile Val Glu Asp Leu Val
145                 150                 155                 160

Met Val Leu Thr Leu Val Leu Leu Pro Ala Val Ala Gly Met Met Glu
                165                 170                 175

Gln Gly Asp Val Gly Phe Ala Thr Leu Ala Val Asp Met Gly Ile Thr
            180                 185                 190

Ile Gly Lys Val Ile Ala Phe Ile Ala Ile Met Met Leu Val Gly Arg
        195                 200                 205

Arg Leu Val Pro Trp Ile Met Ala Arg Ser Ala Ala Thr Gly Ser Arg
    210                 215                 220

Glu Leu Phe Thr Leu Ser Val Leu Ala Leu Ala Leu Gly Ile Ala Phe
225                 230                 235                 240

Gly Ala Val Glu Leu Phe Asp Val Ser Phe Ala Leu Gly Ala Phe Phe
                245                 250                 255

Ala Gly Met Val Leu Asn Glu Ser Glu Leu Ser His Arg Ala Ala His
            260                 265                 270

Asp Thr Leu Pro Leu Arg Asp Ala Phe Ala Val Leu Phe Phe Val Ser
        275                 280                 285

Val Gly Met Leu Phe Asp Pro Leu Ile Leu Ile Gln Gln Pro Leu Ala

290                 295                 300

Val Leu Ala Thr Leu Ala Ile Ile Leu Phe Gly Lys Ser Val Ala Ala
305                 310                 315                 320

Leu Phe Leu Val Arg Leu Phe Gly His Ser Gln Arg Thr Ala Leu Thr
                325                 330                 335

Ile Ala Ala Ser Leu Ala Gln Ile Gly Glu Phe Ala Phe Ile Leu Ala
            340                 345                 350

Gly Leu Gly Met Ala Leu Asn Leu Leu Pro Gln Ala Gly Gln Asn Leu
        355                 360                 365

Val Leu Ala Gly Ala Ile Leu Ser Ile Met Leu Asn Pro Val Leu Phe
    370                 375                 380

Ala Leu Leu Glu Lys Tyr Leu Ala Lys Thr Glu Thr Leu Glu Glu Gln
385                 390                 395                 400

Thr Leu Glu Glu Ala Ile Glu Glu Glu Lys Gln Ile Pro Val Asp Ile
                405                 410                 415

Cys Asn His Ala Leu Leu Val Gly Tyr Gly Arg Val Gly Ser Leu Leu
            420                 425                 430

Gly Glu Lys Leu Leu Ala Ser Asp Ile Pro Leu Val Val Ile Glu Thr
        435                 440                 445

Ser Arg Thr Arg Val Asp Glu Leu Arg Glu Arg Gly Val Arg Ala Val
    450                 455                 460

Leu Gly Asn Ala Ala Asn Glu Glu Ile Met Gln Leu Ala His Leu Glu
465                 470                 475                 480

Cys Ala Lys Trp Leu Ile Leu Thr Ile Pro Asn Gly Tyr Glu Ala Gly
                485                 490                 495

Glu Ile Val Ala Ser Ala Arg Ala Lys Asn Pro Asp Ile Glu Ile Ile
            500                 505                 510

Ala Arg Ala His Tyr Asp Asp Glu Val Ala Tyr Ile Thr Glu Arg Gly
        515                 520                 525

Ala Asn Gln Val Val Met Gly Glu Arg Glu Ile Ala Arg Thr Met Leu
    530                 535                 540

Glu Leu Leu Glu Thr Pro Pro Ala Gly Glu Val Val Thr Arg
545                 550                 555

<210>  7
<211>  345
<212>  DNA
<213>  Escherichia coli K12

<220>
<221>  CDS
<222>  (1)..(342)
<223>  ygaC

<400>  7
atg tat tta cga cca gac gag gtg gcg cgc gta ctt gaa aaa gtc ggt        48
Met Tyr Leu Arg Pro Asp Glu Val Ala Arg Val Leu Glu Lys Val Gly
1               5                   10                  15

ttt act gtc gat gtg gta acc cag aaa gcg tat ggt tac cgc cgt ggg        96
Phe Thr Val Asp Val Val Thr Gln Lys Ala Tyr Gly Tyr Arg Arg Gly
            20                  25                  30

gaa aat tat gtc tat gtt aat cgc gaa gcg cga atg gga cgt acc gct       144
Glu Asn Tyr Val Tyr Val Asn Arg Glu Ala Arg Met Gly Arg Thr Ala
        35                  40                  45

ctg gta att cat cca aca tta aaa gaa cgc agt tcg aca ctg gct gaa       192
Leu Val Ile His Pro Thr Leu Lys Glu Arg Ser Ser Thr Leu Ala Glu
    50                  55                  60

ccc gct tcc gat att aaa act tgc gat cat tac cag caa ttt ccg ctc       240
Pro Ala Ser Asp Ile Lys Thr Cys Asp His Tyr Gln Gln Phe Pro Leu
65                  70                  75                  80

tat tta gca ggc gag cga cac gag cat tac ggt atc ccg cat ggc ttt       288
Tyr Leu Ala Gly Glu Arg His Glu His Tyr Gly Ile Pro His Gly Phe
                85                  90                  95

agt tcg cgt gtt gcg ctt gaa cgt tat ttg aat ggt tta ttt ggc gaa       336
Ser Ser Arg Val Ala Leu Glu Arg Tyr Leu Asn Gly Leu Phe Gly Glu
            100                 105                 110

gcc agt taa                                                           345
Ala Ser

<210>  8
<211>  114
<212>  PRT
<213>  Escherichia coli K12

<400>  8

Met Tyr Leu Arg Pro Asp Glu Val Ala Arg Val Leu Glu Lys Val Gly
1               5                   10                  15

Phe Thr Val Asp Val Val Thr Gln Lys Ala Tyr Gly Tyr Arg Arg Gly
            20                  25                  30

Glu Asn Tyr Val Tyr Val Asn Arg Glu Ala Arg Met Gly Arg Thr Ala
        35                  40                  45

Leu Val Ile His Pro Thr Leu Lys Glu Arg Ser Ser Thr Leu Ala Glu
    50                  55                  60

Pro Ala Ser Asp Ile Lys Thr Cys Asp His Tyr Gln Gln Phe Pro Leu
65                  70                  75                  80

Tyr Leu Ala Gly Glu Arg His Glu His Tyr Gly Ile Pro His Gly Phe
                85                  90                  95

Ser Ser Arg Val Ala Leu Glu Arg Tyr Leu Asn Gly Leu Phe Gly Glu
            100                 105                 110

Ala Ser

<210>  9
<211>  345
<212>  DNA
<213>  Escherichia coli O157:H7

<220>
<221>  CDS
<222>  (1)..(342)
<223>  ygaC

<400>  9
atg tat tta cga cca gac gag gtg gcg cgc gta ctt gaa aaa gtc ggt        48
Met Tyr Leu Arg Pro Asp Glu Val Ala Arg Val Leu Glu Lys Val Gly
1               5                   10                  15

ttt act gtc gat gtg gta acc cag aaa gcg tat ggt tac cgc cgt ggg        96
Phe Thr Val Asp Val Val Thr Gln Lys Ala Tyr Gly Tyr Arg Arg Gly
            20                  25                  30

gaa aat tat gtc tat gtt aat cgc gaa gcg cga atg gga cgt acc gct       144
Glu Asn Tyr Val Tyr Val Asn Arg Glu Ala Arg Met Gly Arg Thr Ala
        35                  40                  45

ctg gta att cat cca aca tta aaa gaa cgc agt tcg aca ctg gct gaa       192
Leu Val Ile His Pro Thr Leu Lys Glu Arg Ser Ser Thr Leu Ala Glu
    50                  55                  60

ccc gct tcc gat att aaa act tgc gat cat tac cag caa ttt ccg ctc       240
Pro Ala Ser Asp Ile Lys Thr Cys Asp His Tyr Gln Gln Phe Pro Leu
65                  70                  75                  80

tat tta gca ggc gag cga cac gag cat tac ggt atc ccg cat ggc ttt       288
Tyr Leu Ala Gly Glu Arg His Glu His Tyr Gly Ile Pro His Gly Phe
                85                  90                  95

agt tcg cgt gtt gcg ctt gaa cgt tat ttg aat ggt tta ttt ggc gaa       336
Ser Ser Arg Val Ala Leu Glu Arg Tyr Leu Asn Gly Leu Phe Gly Glu
            100                 105                 110

gcc agt taa                                                           345
Ala Ser

<210>  10
<211>  114
<212>  PRT
<213>  Escherichia coli O157:H7

<400>  10

Met Tyr Leu Arg Pro Asp Glu Val Ala Arg Val Leu Glu Lys Val Gly
1               5                   10                  15

Phe Thr Val Asp Val Val Thr Gln Lys Ala Tyr Gly Tyr Arg Arg Gly
            20                  25                  30

Glu Asn Tyr Val Tyr Val Asn Arg Glu Ala Arg Met Gly Arg Thr Ala
        35                  40                  45

Leu Val Ile His Pro Thr Leu Lys Glu Arg Ser Ser Thr Leu Ala Glu
    50                  55                  60

Pro Ala Ser Asp Ile Lys Thr Cys Asp His Tyr Gln Gln Phe Pro Leu
65                  70                  75                  80

Tyr Leu Ala Gly Glu Arg His Glu His Tyr Gly Ile Pro His Gly Phe
                85                  90                  95

Ser Ser Arg Val Ala Leu Glu Arg Tyr Leu Asn Gly Leu Phe Gly Glu
            100                 105                 110

Ala Ser

<210>  11
<211>  399
<212>  DNA
<213>  Escherichia coli K12

<220>
<221>  CDS
<222>  (1)..(396)
<223>  yohJ

<400>  11
atg agt aag aca ctg aac att atc tgg caa tat tta cgc gct ttc gtc       48
Met Ser Lys Thr Leu Asn Ile Ile Trp Gln Tyr Leu Arg Ala Phe Val
1               5                   10                  15

ctg att tat gcc tgc ctg tat gca ggc att ttc att gct tcc ctg cta       96
Leu Ile Tyr Ala Cys Leu Tyr Ala Gly Ile Phe Ile Ala Ser Leu Leu
            20                  25                  30

ccg gta acc att ccg ggc agc atc atc ggg atg ctg atc ctg ttt gtc      144
Pro Val Thr Ile Pro Gly Ser Ile Ile Gly Met Leu Ile Leu Phe Val
        35                  40                  45

ctg ctg gcc ttg caa att ctt ccg gca aaa tgg gtc aat ccg ggg tgc      192

Leu Leu Ala Leu Gln Ile Leu Pro Ala Lys Trp Val Asn Pro Gly Cys
    50                  55                  60

tac gta ctg att cgc tat atg gcg cta ttg ttt gtg ccg att ggc gta       240
Tyr Val Leu Ile Arg Tyr Met Ala Leu Leu Phe Val Pro Ile Gly Val
65                  70                  75                  80

ggc gtc atg caa tat ttt gat ttg ctc cgc gca cag ttt ggc ccg gta       288
Gly Val Met Gln Tyr Phe Asp Leu Leu Arg Ala Gln Phe Gly Pro Val
                85                  90                  95

gtg gtt tcc tgt gca gtc agt acg ctg gtg gtt ttt ctg gtg gtg agc       336
Val Val Ser Cys Ala Val Ser Thr Leu Val Val Phe Leu Val Val Ser
            100                 105                 110

tgg agt tcg caa ctg gta cac ggt gaa cgt aaa gtc gta ggt cag aaa       384
Trp Ser Ser Gln Leu Val His Gly Glu Arg Lys Val Val Gly Gln Lys
        115                 120                 125

gga tca gaa gaa tga                                                   399
Gly Ser Glu Glu
    130


<210>  12
<211>  132
<212>  PRT
<213>  Escherichia coli K12

<400>  12

Met Ser Lys Thr Leu Asn Ile Ile Trp Gln Tyr Leu Arg Ala Phe Val
1               5                   10                  15


Leu Ile Tyr Ala Cys Leu Tyr Ala Gly Ile Phe Ile Ala Ser Leu Leu
            20                  25                  30


Pro Val Thr Ile Pro Gly Ser Ile Ile Gly Met Leu Ile Leu Phe Val
        35                  40                  45


Leu Leu Ala Leu Gln Ile Leu Pro Ala Lys Trp Val Asn Pro Gly Cys
    50                  55                  60


Tyr Val Leu Ile Arg Tyr Met Ala Leu Leu Phe Val Pro Ile Gly Val
65                  70                  75                  80


Gly Val Met Gln Tyr Phe Asp Leu Leu Arg Ala Gln Phe Gly Pro Val
                85                  90                  95


Val Val Ser Cys Ala Val Ser Thr Leu Val Val Phe Leu Val Val Ser
            100                 105                 110


Trp Ser Ser Gln Leu Val His Gly Glu Arg Lys Val Val Gly Gln Lys
        115                 120                 125


Gly Ser Glu Glu
    130

<210>  13
<211>  399
<212>  DNA
<213>  Escherichia coli O157:H7

<220>
<221>  CDS
<222>  (1)..(396)
<223>  yohJ

<400>  13
atg agt aag aca ctg aac att atc tgg caa tat tta cgc gct ttc gtc       48
Met Ser Lys Thr Leu Asn Ile Ile Trp Gln Tyr Leu Arg Ala Phe Val
1               5                   10                  15

ctg att tat gcc tgc ctg tat gca ggc att ttc att gct tcc ctg cta       96
Leu Ile Tyr Ala Cys Leu Tyr Ala Gly Ile Phe Ile Ala Ser Leu Leu
            20                  25                  30

ccg gta acc att ccg ggc agc atc atc ggg atg ctg atc ctg ttt gtc      144
Pro Val Thr Ile Pro Gly Ser Ile Ile Gly Met Leu Ile Leu Phe Val
        35                  40                  45

ctg ctg gcc ttg caa att ctt ccg gcg aaa tgg gtc aat ccg ggg tgc      192
Leu Leu Ala Leu Gln Ile Leu Pro Ala Lys Trp Val Asn Pro Gly Cys
    50                  55                  60

tac gta ctg att cgc tat atg gcg cta ttg ttt gtg ccg att ggc gta      240
Tyr Val Leu Ile Arg Tyr Met Ala Leu Leu Phe Val Pro Ile Gly Val
65                  70                  75                  80

ggc gtc atg caa tat ttt gat ttg ctc cgc gca cag ttt ggc ccg gta      288
Gly Val Met Gln Tyr Phe Asp Leu Leu Arg Ala Gln Phe Gly Pro Val
                85                  90                  95

gtg gtt tcc tgt gca atc agt acg ctg gtg gtt ttt ctg gtg gtg agc      336
Val Val Ser Cys Ala Ile Ser Thr Leu Val Val Phe Leu Val Val Ser
            100                 105                 110

tgg agt tcg caa ctg gta cac ggt gaa cgt aaa gtc gta ggt cag aaa      384
Trp Ser Ser Gln Leu Val His Gly Glu Arg Lys Val Val Gly Gln Lys
        115                 120                 125

gga tca gaa gaa tga                                                  399
Gly Ser Glu Glu
    130


<210>  14
<211>  132
<212>  PRT
<213>  Escherichia coli O157:H7

<400>  14

Met Ser Lys Thr Leu Asn Ile Ile Trp Gln Tyr Leu Arg Ala Phe Val
1               5                   10                  15


Leu Ile Tyr Ala Cys Leu Tyr Ala Gly Ile Phe Ile Ala Ser Leu Leu
            20                  25                  30

Pro Val Thr Ile Pro Gly Ser Ile Ile Gly Met Leu Ile Leu Phe Val
        35                  40                  45

Leu Leu Ala Leu Gln Ile Leu Pro Ala Lys Trp Val Asn Pro Gly Cys
    50                  55                  60

Tyr Val Leu Ile Arg Tyr Met Ala Leu Leu Phe Val Pro Ile Gly Val
65                  70                  75                  80

Gly Val Met Gln Tyr Phe Asp Leu Leu Arg Ala Gln Phe Gly Pro Val
                85                  90                  95

Val Val Ser Cys Ala Ile Ser Thr Leu Val Val Phe Leu Val Val Ser
            100                 105                 110

Trp Ser Ser Gln Leu Val His Gly Glu Arg Lys Val Val Gly Gln Lys
        115                 120                 125

Gly Ser Glu Glu
    130

<210>  15
<211>  1101
<212>  DNA
<213>  Escherichia coli K12

<220>
<221>  CDS
<222>  (1)..(1098)
<223>  yjgP

<400>  15
gtg ata atc ata aga tat ctg gtg cgg gag acg ctc aaa agc cag ctg       48
Val Ile Ile Ile Arg Tyr Leu Val Arg Glu Thr Leu Lys Ser Gln Leu
1               5                   10                  15

gcg ata ctc ttc atc ttg ctt ttg atc ttc ttc tgt caa aag tta gtg       96
Ala Ile Leu Phe Ile Leu Leu Leu Ile Phe Phe Cys Gln Lys Leu Val
            20                  25                  30

agg atc ctc ggc gca gcg gtt gac ggc gat att ccg gcg aat ctg gtg      144
Arg Ile Leu Gly Ala Ala Val Asp Gly Asp Ile Pro Ala Asn Leu Val
        35                  40                  45

ctc tcc ctt ctc ggg ttg ggc gtg ccg gaa atg gcg cag ctt atc ctg      192
Leu Ser Leu Leu Gly Leu Gly Val Pro Glu Met Ala Gln Leu Ile Leu
    50                  55                  60

cca tta agc ctg ttc ctc ggg ctg ctg atg acg ctg ggc aaa ctg tat      240
Pro Leu Ser Leu Phe Leu Gly Leu Leu Met Thr Leu Gly Lys Leu Tyr
65                  70                  75                  80

acc gaa agt gaa att acg gta atg cat gcc tgc ggc ctg agc aaa gcg      288
Thr Glu Ser Glu Ile Thr Val Met His Ala Cys Gly Leu Ser Lys Ala
                85                  90                  95

gtt ctg gtg aaa gcg gca atg atc ctt gcg gta ttc acg gca atc gta     336
Val Leu Val Lys Ala Ala Met Ile Leu Ala Val Phe Thr Ala Ile Val
            100                 105                 110

gcg gcg gtt aac gtg atg tgg gcg gga ccg tgg tca tcg cgt cat cag     384
Ala Ala Val Asn Val Met Trp Ala Gly Pro Trp Ser Ser Arg His Gln
        115                 120                 125

gat gaa gtg tta gca gaa gcg aaa gcg aac cct ggc atg gcg gcg ctg     432
Asp Glu Val Leu Ala Glu Ala Lys Ala Asn Pro Gly Met Ala Ala Leu
    130                 135                 140

gcg caa ggg caa ttc cag caa gcg act aat ggc agc tcg gtg ctg ttc     480
Ala Gln Gly Gln Phe Gln Gln Ala Thr Asn Gly Ser Ser Val Leu Phe
145                 150                 155                 160

atc gaa agc gtt gac ggc agc gat ttc aaa gat gtg ttc ctc gcg caa     528
Ile Glu Ser Val Asp Gly Ser Asp Phe Lys Asp Val Phe Leu Ala Gln
                165                 170                 175

att cga cca aaa ggt aat gca cgt cct tct gtg gtg gtg gcc gat tcc     576
Ile Arg Pro Lys Gly Asn Ala Arg Pro Ser Val Val Val Ala Asp Ser
            180                 185                 190

gga cat tta acc cag ctg cgc gac ggc tcc cag gtc gtc act ctc aac     624
Gly His Leu Thr Gln Leu Arg Asp Gly Ser Gln Val Val Thr Leu Asn
        195                 200                 205

cag gga acg cgc ttc gaa ggc act gca ttg tta cgt gat ttc cgc att     672
Gln Gly Thr Arg Phe Glu Gly Thr Ala Leu Leu Arg Asp Phe Arg Ile
    210                 215                 220

acg gac ttc cag gat tat cag gcg atc att ggt cac cag gcg gtg gcg     720
Thr Asp Phe Gln Asp Tyr Gln Ala Ile Ile Gly His Gln Ala Val Ala
225                 230                 235                 240

ctc gac ccg aac gat acc gac cag atg gac atg cgc aca ttg tgg aac     768
Leu Asp Pro Asn Asp Thr Asp Gln Met Asp Met Arg Thr Leu Trp Asn
                245                 250                 255

act gac acc gat cgt gct cgc gca gaa ctg aac tgg cgt atc acg ttg     816
Thr Asp Thr Asp Arg Ala Arg Ala Glu Leu Asn Trp Arg Ile Thr Leu
            260                 265                 270

gta ttc acc gtg ttt atg atg gca ctt atg gtc gta ccg ctg agc gtg     864
Val Phe Thr Val Phe Met Met Ala Leu Met Val Val Pro Leu Ser Val
        275                 280                 285

gtt aac cca cgt cag gga cgc gta ctg tcg atg ctg cca gcc atg ctg     912
Val Asn Pro Arg Gln Gly Arg Val Leu Ser Met Leu Pro Ala Met Leu
    290                 295                 300

ctg tat cta ctt ttc ttc ctg atc cag acc tcc ctg aaa tcg aac ggc     960
Leu Tyr Leu Leu Phe Phe Leu Ile Gln Thr Ser Leu Lys Ser Asn Gly
305                 310                 315                 320

ggt aaa ggt aag ctg gac ccg acg ctg tgg atg tgg acc gtt aac ctg    1008
Gly Lys Gly Lys Leu Asp Pro Thr Leu Trp Met Trp Thr Val Asn Leu
                325                 330                 335

att tat ctg gct tta gcg att gtt ctc aac ctt tgg gac acc gtg ccg    1056
Ile Tyr Leu Ala Leu Ala Ile Val Leu Asn Leu Trp Asp Thr Val Pro
            340                 345                 350

gtc cgc cgc ctg cgc gcc agt ttt tcg cgt aaa gga gcg gtg tga        1101

Val Arg Arg Leu Arg Ala Ser Phe Ser Arg Lys Gly Ala Val
        355                 360                 365

<210>  16
<211>  366
<212>  PRT
<213>  Escherichia coli K12

<400>  16

Val Ile Ile Ile Arg Tyr Leu Val Arg Glu Thr Leu Lys Ser Gln Leu
1               5                   10                  15

Ala Ile Leu Phe Ile Leu Leu Leu Ile Phe Phe Cys Gln Lys Leu Val
            20                  25                  30

Arg Ile Leu Gly Ala Ala Val Asp Gly Asp Ile Pro Ala Asn Leu Val
        35                  40                  45

Leu Ser Leu Leu Gly Leu Gly Val Pro Glu Met Ala Gln Leu Ile Leu
    50                  55                  60

Pro Leu Ser Leu Phe Leu Gly Leu Leu Met Thr Leu Gly Lys Leu Tyr
65                  70                  75                  80

Thr Glu Ser Glu Ile Thr Val Met His Ala Cys Gly Leu Ser Lys Ala
                85                  90                  95

Val Leu Val Lys Ala Ala Met Ile Leu Ala Val Phe Thr Ala Ile Val
            100                 105                 110

Ala Ala Val Asn Val Met Trp Ala Gly Pro Trp Ser Ser Arg His Gln
        115                 120                 125

Asp Glu Val Leu Ala Glu Ala Lys Ala Asn Pro Gly Met Ala Ala Leu
    130                 135                 140

Ala Gln Gly Gln Phe Gln Gln Ala Thr Asn Gly Ser Ser Val Leu Phe
145                 150                 155                 160

Ile Glu Ser Val Asp Gly Ser Asp Phe Lys Asp Val Phe Leu Ala Gln
                165                 170                 175

Ile Arg Pro Lys Gly Asn Ala Arg Pro Ser Val Val Val Ala Asp Ser
            180                 185                 190

Gly His Leu Thr Gln Leu Arg Asp Gly Ser Gln Val Val Thr Leu Asn
        195                 200                 205

Gln Gly Thr Arg Phe Glu Gly Thr Ala Leu Leu Arg Asp Phe Arg Ile
    210                 215                 220

Thr Asp Phe Gln Asp Tyr Gln Ala Ile Ile Gly His Gln Ala Val Ala
225                 230                 235                 240

Leu Asp Pro Asn Asp Thr Asp Gln Met Asp Met Arg Thr Leu Trp Asn
                245                 250                 255

Thr Asp Thr Asp Arg Ala Arg Ala Glu Leu Asn Trp Arg Ile Thr Leu
            260                 265                 270

Val Phe Thr Val Phe Met Met Ala Leu Met Val Val Pro Leu Ser Val
        275                 280                 285

Val Asn Pro Arg Gln Gly Arg Val Leu Ser Met Leu Pro Ala Met Leu
    290                 295                 300

Leu Tyr Leu Leu Phe Phe Leu Ile Gln Thr Ser Leu Lys Ser Asn Gly
305                 310                 315                 320

Gly Lys Gly Lys Leu Asp Pro Thr Leu Trp Met Trp Thr Val Asn Leu
                325                 330                 335

Ile Tyr Leu Ala Leu Ala Ile Val Leu Asn Leu Trp Asp Thr Val Pro
            340                 345                 350

Val Arg Arg Leu Arg Ala Ser Phe Ser Arg Lys Gly Ala Val
        355                 360                 365

<210>  17
<211>  1101
<212>  DNA
<213>  Escherichia coli O157:H7

<220>
<221>  CDS
<222>  (1)..(1098)
<223>  yjgP

<400>  17
gtg ata atc ata aga tat ctg gtg cgg gag acg ctc aaa agc cag ctg       48
Val Ile Ile Ile Arg Tyr Leu Val Arg Glu Thr Leu Lys Ser Gln Leu
1               5                   10                  15

gcg ata ctc ttc atc ttg ctt ttg atc ttc ttc tgt caa aag tta gtg       96
Ala Ile Leu Phe Ile Leu Leu Leu Ile Phe Phe Cys Gln Lys Leu Val
            20                  25                  30

agg atc ctc ggc gca gcg gtt gac ggc gat att ccg gcg aat ctg gtg      144
Arg Ile Leu Gly Ala Ala Val Asp Gly Asp Ile Pro Ala Asn Leu Val
        35                  40                  45

ctc tcc ctt ctc ggg ttg ggc gtg ccg gaa atg gcg cag ctt atc ctg      192
Leu Ser Leu Leu Gly Leu Gly Val Pro Glu Met Ala Gln Leu Ile Leu
    50                  55                  60

cca tta agc ctg ttc ctc ggg ctg ctg atg acg ctg ggc aaa ctg tat 240
Pro Leu Ser Leu Phe Leu Gly Leu Leu Met Thr Leu Gly Lys Leu Tyr
65                  70                  75                  80

acc gaa agt gaa att acg gta atg cat gcc tgc ggc ctg agc aaa gcg 288
Thr Glu Ser Glu Ile Thr Val Met His Ala Cys Gly Leu Ser Lys Ala
                85                  90                  95

gtt ctg gtg aaa gcg gca atg atc ctt gcg gta ttc acg gca atc gtc 336
Val Leu Val Lys Ala Ala Met Ile Leu Ala Val Phe Thr Ala Ile Val
            100                 105                 110

gcg gcg gtt aac gtg atg tgg gcg gga ccg tgg tca tcg cgt cat cag 384
Ala Ala Val Asn Val Met Trp Ala Gly Pro Trp Ser Ser Arg His Gln
        115                 120                 125

gat gaa gtg tta gca gaa gcg aaa gcg aac cct ggc atg gcg gcg ctg 432
Asp Glu Val Leu Ala Glu Ala Lys Ala Asn Pro Gly Met Ala Ala Leu
    130                 135                 140

gcg caa ggg caa ttc cag caa gcg act aat ggc agt tcg gtg ctg ttc 480
Ala Gln Gly Gln Phe Gln Gln Ala Thr Asn Gly Ser Ser Val Leu Phe
145                 150                 155                 160

atc gaa agc gtt gac ggc agc gat ttc aaa gat gtg ttc ctc gcg caa 528
Ile Glu Ser Val Asp Gly Ser Asp Phe Lys Asp Val Phe Leu Ala Gln
                165                 170                 175

att cga cca aaa ggt aat gca cgt cct tct gtg gtg gtg gcc gat tcc 576
Ile Arg Pro Lys Gly Asn Ala Arg Pro Ser Val Val Val Ala Asp Ser
            180                 185                 190

gga cat tta acc cag ctg cgc gac ggc tcc cag gtc gtc act ctc aac 624
Gly His Leu Thr Gln Leu Arg Asp Gly Ser Gln Val Val Thr Leu Asn
        195                 200                 205

cag gga acg cgc ttc gaa ggc act gca ctg tta cgt gat ttc cgc att 672
Gln Gly Thr Arg Phe Glu Gly Thr Ala Leu Leu Arg Asp Phe Arg Ile
    210                 215                 220

acg gat ttc cag gat tat cag gcg atc att ggt cac cag gcg gtg gcg 720
Thr Asp Phe Gln Asp Tyr Gln Ala Ile Ile Gly His Gln Ala Val Ala
225                 230                 235                 240

ctc gac ccg aac gat acc gac cag atg gac atg cgc aca ttg tgg aac 768
Leu Asp Pro Asn Asp Thr Asp Gln Met Asp Met Arg Thr Leu Trp Asn
                245                 250                 255

act gac acc gat cgt gct cgc gca gaa ctg aac tgg cgt atc acg ttg 816
Thr Asp Thr Asp Arg Ala Arg Ala Glu Leu Asn Trp Arg Ile Thr Leu
            260                 265                 270

gta ttc acc gtg ttt atg atg gca ctt atg gtc gta ccg ctg agc gtg 864
Val Phe Thr Val Phe Met Met Ala Leu Met Val Val Pro Leu Ser Val
        275                 280                 285

gtt aac cca cgt cag gga cgc gta ctg tcg atg ctg cca gcc atg ctg 912
Val Asn Pro Arg Gln Gly Arg Val Leu Ser Met Leu Pro Ala Met Leu
    290                 295                 300

ctg tat cta ctt ttc ttc ctg atc cag acc tcc ctg aaa tcg aac ggc 960
Leu Tyr Leu Leu Phe Phe Leu Ile Gln Thr Ser Leu Lys Ser Asn Gly
305                 310                 315                 320

ggt aaa ggt aag ctg gac ccg acg ctg tgg atg tgg acc gtt aac ctg      1008
Gly Lys Gly Lys Leu Asp Pro Thr Leu Trp Met Trp Thr Val Asn Leu
                325                 330                 335

att tat ctg gct ttg gcg att gtt ctc aac ctt tgg gac acc gtg ccg      1056
Ile Tyr Leu Ala Leu Ala Ile Val Leu Asn Leu Trp Asp Thr Val Pro
            340                 345                 350

gtc cgc cgc ctg cgc gcc agt ttt tcg cgt aaa gga gcg gtg tga          1101
Val Arg Arg Leu Arg Ala Ser Phe Ser Arg Lys Gly Ala Val
        355                 360                 365

<210>  18
<211>  366
<212>  PRT
<213>  Escherichia coli O157:H7

<400>  18

Val Ile Ile Ile Arg Tyr Leu Val Arg Glu Thr Leu Lys Ser Gln Leu
1               5                   10                  15

Ala Ile Leu Phe Ile Leu Leu Leu Ile Phe Phe Cys Gln Lys Leu Val
            20                  25                  30

Arg Ile Leu Gly Ala Ala Val Asp Gly Asp Ile Pro Ala Asn Leu Val
        35                  40                  45

Leu Ser Leu Leu Gly Leu Gly Val Pro Glu Met Ala Gln Leu Ile Leu
    50                  55                  60

Pro Leu Ser Leu Phe Leu Gly Leu Leu Met Thr Leu Gly Lys Leu Tyr
65                  70                  75                  80

Thr Glu Ser Glu Ile Thr Val Met His Ala Cys Gly Leu Ser Lys Ala
                85                  90                  95

Val Leu Val Lys Ala Ala Met Ile Leu Ala Val Phe Thr Ala Ile Val
            100                 105                 110

Ala Ala Val Asn Val Met Trp Ala Gly Pro Trp Ser Ser Arg His Gln
        115                 120                 125

Asp Glu Val Leu Ala Glu Ala Lys Ala Asn Pro Gly Met Ala Ala Leu
    130                 135                 140

Ala Gln Gly Gln Phe Gln Gln Ala Thr Asn Gly Ser Ser Val Leu Phe
145                 150                 155                 160

Ile Glu Ser Val Asp Gly Ser Asp Phe Lys Asp Val Phe Leu Ala Gln
                165                 170                 175

Ile Arg Pro Lys Gly Asn Ala Arg Pro Ser Val Val Val Ala Asp Ser

180                 185                 190

Gly His Leu Thr Gln Leu Arg Asp Gly Ser Gln Val Val Thr Leu Asn
        195                 200                 205

Gln Gly Thr Arg Phe Glu Gly Thr Ala Leu Leu Arg Asp Phe Arg Ile
    210                 215                 220

Thr Asp Phe Gln Asp Tyr Gln Ala Ile Ile Gly His Gln Ala Val Ala
225                 230                 235                 240

Leu Asp Pro Asn Asp Thr Asp Gln Met Asp Met Arg Thr Leu Trp Asn
                245                 250                 255

Thr Asp Thr Asp Arg Ala Arg Ala Glu Leu Asn Trp Arg Ile Thr Leu
            260                 265                 270

Val Phe Thr Val Phe Met Met Ala Leu Met Val Val Pro Leu Ser Val
        275                 280                 285

Val Asn Pro Arg Gln Gly Arg Val Leu Ser Met Leu Pro Ala Met Leu
    290                 295                 300

Leu Tyr Leu Leu Phe Phe Leu Ile Gln Thr Ser Leu Lys Ser Asn Gly
305                 310                 315                 320

Gly Lys Gly Lys Leu Asp Pro Thr Leu Trp Met Trp Thr Val Asn Leu
                325                 330                 335

Ile Tyr Leu Ala Leu Ala Ile Val Leu Asn Leu Trp Asp Thr Val Pro
            340                 345                 350

Val Arg Arg Leu Arg Ala Ser Phe Ser Arg Lys Gly Ala Val
        355                 360                 365

<210>  19
<211>  1137
<212>  DNA
<213>  Escherichia coli K12

<220>
<221>  CDS
<222>  (1)..(1134)
<223>  yggW

<400>  19
atg gtt aaa tta cct ccg ctg agt ctc tac att cac atc ccg tgg tgc       48
Met Val Lys Leu Pro Pro Leu Ser Leu Tyr Ile His Ile Pro Trp Cys
1               5                   10                  15

gtg cag aaa tgc ccg tac tgc gat ttc aac tct cac gcg ttg aaa gga       96
Val Gln Lys Cys Pro Tyr Cys Asp Phe Asn Ser His Ala Leu Lys Gly

20                  25                  30

gaa gtg ccg cac gac gat tat gtt cag cat ctg ctt aac gat ctg gac      144
Glu Val Pro His Asp Asp Tyr Val Gln His Leu Leu Asn Asp Leu Asp
        35                  40                  45

aac gat gtg gct tac gct cag ggc cgt gaa gta aag aca atc ttt att      192
Asn Asp Val Ala Tyr Ala Gln Gly Arg Glu Val Lys Thr Ile Phe Ile
    50                  55                  60

ggc ggt ggt acg ccg agc ctg ctt tcc ggc ccg gcg atg caa acg ctg      240
Gly Gly Gly Thr Pro Ser Leu Leu Ser Gly Pro Ala Met Gln Thr Leu
65                  70                  75                  80

ctg gac ggc gtg cgt gcg cgt ttg ccg ctg gca gcg gat gca gaa att      288
Leu Asp Gly Val Arg Ala Arg Leu Pro Leu Ala Ala Asp Ala Glu Ile
                85                  90                  95

act atg gaa gcg aac cct ggc acg gta gaa gcc gat cgc ttt gtc gat      336
Thr Met Glu Ala Asn Pro Gly Thr Val Glu Ala Asp Arg Phe Val Asp
            100                 105                 110

tat cag cgt gct ggt gtg aac cgc atc tct att ggt gtg cag agt ttt      384
Tyr Gln Arg Ala Gly Val Asn Arg Ile Ser Ile Gly Val Gln Ser Phe
        115                 120                 125

agc gaa gaa aag ctg aaa cga ctt ggg cgt att cat ggc ccg caa gaa      432
Ser Glu Glu Lys Leu Lys Arg Leu Gly Arg Ile His Gly Pro Gln Glu
    130                 135                 140

gcg aaa cgc gcg gcg aag ctg gcg agc ggt tta ggg tta cgt agc ttt      480
Ala Lys Arg Ala Ala Lys Leu Ala Ser Gly Leu Gly Leu Arg Ser Phe
145                 150                 155                 160

aac ctt gat ttg atg cat ggg ctg ccg gat caa tca ctg gaa gag gcg      528
Asn Leu Asp Leu Met His Gly Leu Pro Asp Gln Ser Leu Glu Glu Ala
                165                 170                 175

ctt ggc gat cta cgc cag gcc att gaa ctg aat ccg ccg cat ctt tcc      576
Leu Gly Asp Leu Arg Gln Ala Ile Glu Leu Asn Pro Pro His Leu Ser
            180                 185                 190

tgg tat caa ctg acc atc gaa ccc aat acg ctg ttt ggt tcg cga cca      624
Trp Tyr Gln Leu Thr Ile Glu Pro Asn Thr Leu Phe Gly Ser Arg Pro
        195                 200                 205

ccg gtg ctg ccg gac gat gac gcg ttg tgg gat ata ttc gaa cag ggg      672
Pro Val Leu Pro Asp Asp Asp Ala Leu Trp Asp Ile Phe Glu Gln Gly
    210                 215                 220

cat cag tta tta acc gca gcg ggt tat cag caa tat gaa act tcc gct      720
His Gln Leu Leu Thr Ala Ala Gly Tyr Gln Gln Tyr Glu Thr Ser Ala
225                 230                 235                 240

tac gcc aaa ccc ggt tat cag tgc cag cac aat ctc aac tac tgg cgc      768
Tyr Ala Lys Pro Gly Tyr Gln Cys Gln His Asn Leu Asn Tyr Trp Arg
                245                 250                 255

ttt ggt gac tac atc ggt att ggc tgc ggc gca cac ggc aaa gtg acc      816
Phe Gly Asp Tyr Ile Gly Ile Gly Cys Gly Ala His Gly Lys Val Thr
            260                 265                 270

ttc ccg gat ggg cgc att ctg cgt acc acc aaa acg cgt cat ccg cgt      864
Phe Pro Asp Gly Arg Ile Leu Arg Thr Thr Lys Thr Arg His Pro Arg
        275                 280                 285

ggt ttt atg caa gga agg tat ctg gaa agc cag cgt gat gtc gaa gcc 912
Gly Phe Met Gln Gly Arg Tyr Leu Glu Ser Gln Arg Asp Val Glu Ala
    290                 295                 300

aca gat aag ccg ttt gag ttc ttt atg aat cgc ttc cgt ctg ctg gag 960
Thr Asp Lys Pro Phe Glu Phe Phe Met Asn Arg Phe Arg Leu Leu Glu
305                 310                 315                 320

gcc gcg ccg cgc gtg gag ttt att gcg tat acc ggg ctt tgc gaa gat 1008
Ala Ala Pro Arg Val Glu Phe Ile Ala Tyr Thr Gly Leu Cys Glu Asp
                325                 330                 335

gtg att cgc cca cag tta gac gag gcg att gcc cag ggt tat ctc acc 1056
Val Ile Arg Pro Gln Leu Asp Glu Ala Ile Ala Gln Gly Tyr Leu Thr
            340                 345                 350

gaa tgt gcg gat tac tgg cag ata acg gaa cat ggg aag ctg ttt tta 1104
Glu Cys Ala Asp Tyr Trp Gln Ile Thr Glu His Gly Lys Leu Phe Leu
        355                 360                 365

aat tcg ctg ctg gag ctt ttt ctg gct gag taa 1137
Asn Ser Leu Leu Glu Leu Phe Leu Ala Glu
    370                 375

<210> 20
<211> 378
<212> PRT
<213> Escherichia coli K12

<400> 20

Met Val Lys Leu Pro Pro Leu Ser Leu Tyr Ile His Ile Pro Trp Cys
1               5                   10                  15

Val Gln Lys Cys Pro Tyr Cys Asp Phe Asn Ser His Ala Leu Lys Gly
            20                  25                  30

Glu Val Pro His Asp Asp Tyr Val Gln His Leu Leu Asn Asp Leu Asp
        35                  40                  45

Asn Asp Val Ala Tyr Ala Gln Gly Arg Glu Val Lys Thr Ile Phe Ile
    50                  55                  60

Gly Gly Gly Thr Pro Ser Leu Leu Ser Gly Pro Ala Met Gln Thr Leu
65                  70                  75                  80

Leu Asp Gly Val Arg Ala Arg Leu Pro Leu Ala Ala Asp Ala Glu Ile
                85                  90                  95

Thr Met Glu Ala Asn Pro Gly Thr Val Glu Ala Asp Arg Phe Val Asp
            100                 105                 110

Tyr Gln Arg Ala Gly Val Asn Arg Ile Ser Ile Gly Val Gln Ser Phe
        115                 120                 125

Ser Glu Glu Lys Leu Lys Arg Leu Gly Arg Ile His Gly Pro Gln Glu
    130                 135                 140

Ala Lys Arg Ala Ala Lys Leu Ala Ser Gly Leu Gly Leu Arg Ser Phe
145                 150                 155                 160

Asn Leu Asp Leu Met His Gly Leu Pro Asp Gln Ser Leu Glu Glu Ala
                165                 170                 175

Leu Gly Asp Leu Arg Gln Ala Ile Glu Leu Asn Pro Pro His Leu Ser
            180                 185                 190

Trp Tyr Gln Leu Thr Ile Glu Pro Asn Thr Leu Phe Gly Ser Arg Pro
        195                 200                 205

Pro Val Leu Pro Asp Asp Asp Ala Leu Trp Asp Ile Phe Glu Gln Gly
    210                 215                 220

His Gln Leu Leu Thr Ala Ala Gly Tyr Gln Gln Tyr Glu Thr Ser Ala
225                 230                 235                 240

Tyr Ala Lys Pro Gly Tyr Gln Cys Gln His Asn Leu Asn Tyr Trp Arg
                245                 250                 255

Phe Gly Asp Tyr Ile Gly Ile Gly Cys Gly Ala His Gly Lys Val Thr
            260                 265                 270

Phe Pro Asp Gly Arg Ile Leu Arg Thr Thr Lys Thr Arg His Pro Arg
        275                 280                 285

Gly Phe Met Gln Gly Arg Tyr Leu Glu Ser Gln Arg Asp Val Glu Ala
    290                 295                 300

Thr Asp Lys Pro Phe Glu Phe Phe Met Asn Arg Phe Arg Leu Leu Glu
305                 310                 315                 320

Ala Ala Pro Arg Val Glu Phe Ile Ala Tyr Thr Gly Leu Cys Glu Asp
                325                 330                 335

Val Ile Arg Pro Gln Leu Asp Glu Ala Ile Ala Gln Gly Tyr Leu Thr
            340                 345                 350

Glu Cys Ala Asp Tyr Trp Gln Ile Thr Glu His Gly Lys Leu Phe Leu
        355                 360                 365

Asn Ser Leu Leu Glu Leu Phe Leu Ala Glu
    370                 375

<210>  21

<211>  1137
<212>  DNA
<213>  Escherichia coli O157:H7

<220>
<221>  CDS
<222>  (1)..(1134)
<223>  yggW

<400>  21
atg gtt aaa tta ccg ccg ctg art ctc tac att cac atc ccg tgg tgc        48
Met Val Lys Leu Pro Pro Leu Xaa Leu Tyr Ile His Ile Pro Trp Cys
1               5                   10                  15

gtg cag aaa tgc ccg tac tgc gat ttc aac tct cac gcg ttg aaa gga        96
Val Gln Lys Cys Pro Tyr Cys Asp Phe Asn Ser His Ala Leu Lys Gly
            20                  25                  30

gaa gtg ccg cac gac gat tac gtt cag cat ctg ctt aac gat ctg gac       144
Glu Val Pro His Asp Asp Tyr Val Gln His Leu Leu Asn Asp Leu Asp
        35                  40                  45

aac gat gtg gct tac gct cag ggc cgt gaa gta aag aca atc ttt att       192
Asn Asp Val Ala Tyr Ala Gln Gly Arg Glu Val Lys Thr Ile Phe Ile
    50                  55                  60

ggc ggt ggt acg ccg agc ctg ctt tcc ggc tcg gcg atg caa acg ctg       240
Gly Gly Gly Thr Pro Ser Leu Leu Ser Gly Ser Ala Met Gln Thr Leu
65                  70                  75                  80

ctg gac ggc gtg cgt gcg cgt ttg ccg ctg gca gcg gat gca gaa att       288
Leu Asp Gly Val Arg Ala Arg Leu Pro Leu Ala Ala Asp Ala Glu Ile
                85                  90                  95

acc atg gaa gcg aac ccc ggt acg gta gaa gcc gat cgc ttt gtt gat       336
Thr Met Glu Ala Asn Pro Gly Thr Val Glu Ala Asp Arg Phe Val Asp
            100                 105                 110

tat cag cgt gcc ggt gta aat cgt atc tct att ggg gta cag agt ttt       384
Tyr Gln Arg Ala Gly Val Asn Arg Ile Ser Ile Gly Val Gln Ser Phe
        115                 120                 125

agc gaa gaa aag ctg aaa cgg ctg gga cgt att cat ggc ccg caa gaa       432
Ser Glu Glu Lys Leu Lys Arg Leu Gly Arg Ile His Gly Pro Gln Glu
    130                 135                 140

gcg aaa cga gcg gca aag ctg gcg agc ggt tta ggg tta cgt agc ttt       480
Ala Lys Arg Ala Ala Lys Leu Ala Ser Gly Leu Gly Leu Arg Ser Phe
145                 150                 155                 160

aac ctt gat ttg atg cat ggg cta ccg gat caa tcg ctg gaa gag gcg       528
Asn Leu Asp Leu Met His Gly Leu Pro Asp Gln Ser Leu Glu Glu Ala
                165                 170                 175

ctt ggc gat ctg cgc cag gct att gaa ctg aat ccg ccg cat ctt tcc       576
Leu Gly Asp Leu Arg Gln Ala Ile Glu Leu Asn Pro Pro His Leu Ser
            180                 185                 190

tgg tat caa ctg acc atc gaa cct aat acg ctg ttt ggt tca cgc ccg       624
Trp Tyr Gln Leu Thr Ile Glu Pro Asn Thr Leu Phe Gly Ser Arg Pro
        195                 200                 205

ccg gta ctg ccg gac gac gac gcg ctg tgg gat att ttc gaa cag ggg       672
Pro Val Leu Pro Asp Asp Asp Ala Leu Trp Asp Ile Phe Glu Gln Gly

210                 215                 220

cat cag tta tta acc gca gcg ggt tat cag caa tat gaa act tcc gct        720
His Gln Leu Leu Thr Ala Ala Gly Tyr Gln Gln Tyr Glu Thr Ser Ala
225                 230                 235                 240

tac gcc aaa ccc ggt tat cag tgc cag cac aat ctc aac tac tgg cgc        768
Tyr Ala Lys Pro Gly Tyr Gln Cys Gln His Asn Leu Asn Tyr Trp Arg
                245                 250                 255

ttt ggt gac tac atc ggt att ggc tgc ggc gcg cac ggc aaa gtg acc        816
Phe Gly Asp Tyr Ile Gly Ile Gly Cys Gly Ala His Gly Lys Val Thr
            260                 265                 270

ttc ccg gat ggg cgc att ctg cgt acc acc aaa acg cgt cat ccg cgt        864
Phe Pro Asp Gly Arg Ile Leu Arg Thr Thr Lys Thr Arg His Pro Arg
        275                 280                 285

ggt ttt atg caa gga agg tat ctg gaa agc cag cgt gat gtc gaa gcc        912
Gly Phe Met Gln Gly Arg Tyr Leu Glu Ser Gln Arg Asp Val Glu Ala
    290                 295                 300

gca gat aag ccg ttt gag ttc ttt atg aat cgc ttc cgg ttg ctg gaa        960
Ala Asp Lys Pro Phe Glu Phe Phe Met Asn Arg Phe Arg Leu Leu Glu
305                 310                 315                 320

cct gcg ccg cgc gtg gag ttt agt gcg tat acc ggg ctt tgc gaa gat       1008
Pro Ala Pro Arg Val Glu Phe Ser Ala Tyr Thr Gly Leu Cys Glu Asp
                325                 330                 335

gtg att cgc cca cag tta gac gag gcg att gcc cag ggt tat ctc acc       1056
Val Ile Arg Pro Gln Leu Asp Glu Ala Ile Ala Gln Gly Tyr Leu Thr
            340                 345                 350

gaa tgt gcg gat tac tgg cag att acg gaa cat ggg aag ctg ttt tta       1104
Glu Cys Ala Asp Tyr Trp Gln Ile Thr Glu His Gly Lys Leu Phe Leu
        355                 360                 365

aat tcg ctg ctg gag ctt ttt ctg gct gag taa                           1137
Asn Ser Leu Leu Glu Leu Phe Leu Ala Glu
    370                 375

<210>  22
<211>  378
<212>  PRT
<213>  Escherichia coli O157:H7

<220>
<221>  misc_feature
<222>  (8)..(8)
<223>  The 'Xaa' at location 8 stands for Ser, or Asn.

<400>  22

Met Val Lys Leu Pro Pro Leu Xaa Leu Tyr Ile His Ile Pro Trp Cys
1               5                   10                  15

Val Gln Lys Cys Pro Tyr Cys Asp Phe Asn Ser His Ala Leu Lys Gly
            20                  25                  30

Glu Val Pro His Asp Asp Tyr Val Gln His Leu Leu Asn Asp Leu Asp
        35                  40                  45

Asn Asp Val Ala Tyr Ala Gln Gly Arg Glu Val Lys Thr Ile Phe Ile
    50                  55                  60

Gly Gly Gly Thr Pro Ser Leu Leu Ser Gly Ser Ala Met Gln Thr Leu
65                  70                  75                  80

Leu Asp Gly Val Arg Ala Arg Leu Pro Leu Ala Ala Asp Ala Glu Ile
                85                  90                  95

Thr Met Glu Ala Asn Pro Gly Thr Val Glu Ala Asp Arg Phe Val Asp
            100                 105                 110

Tyr Gln Arg Ala Gly Val Asn Arg Ile Ser Ile Gly Val Gln Ser Phe
        115                 120                 125

Ser Glu Glu Lys Leu Lys Arg Leu Gly Arg Ile His Gly Pro Gln Glu
    130                 135                 140

Ala Lys Arg Ala Ala Lys Leu Ala Ser Gly Leu Gly Leu Arg Ser Phe
145                 150                 155                 160

Asn Leu Asp Leu Met His Gly Leu Pro Asp Gln Ser Leu Glu Glu Ala
                165                 170                 175

Leu Gly Asp Leu Arg Gln Ala Ile Glu Leu Asn Pro Pro His Leu Ser
            180                 185                 190

Trp Tyr Gln Leu Thr Ile Glu Pro Asn Thr Leu Phe Gly Ser Arg Pro
        195                 200                 205

Pro Val Leu Pro Asp Asp Asp Ala Leu Trp Asp Ile Phe Glu Gln Gly
    210                 215                 220

His Gln Leu Leu Thr Ala Ala Gly Tyr Gln Gln Tyr Glu Thr Ser Ala
225                 230                 235                 240

Tyr Ala Lys Pro Gly Tyr Gln Cys Gln His Asn Leu Asn Tyr Trp Arg
                245                 250                 255

Phe Gly Asp Tyr Ile Gly Ile Gly Cys Gly Ala His Gly Lys Val Thr
            260                 265                 270

Phe Pro Asp Gly Arg Ile Leu Arg Thr Thr Lys Thr Arg His Pro Arg
        275                 280                 285

Gly Phe Met Gln Gly Arg Tyr Leu Glu Ser Gln Arg Asp Val Glu Ala
    290                 295                 300

Ala Asp Lys Pro Phe Glu Phe Phe Met Asn Arg Phe Arg Leu Leu Glu
305                 310                 315                 320

Pro Ala Pro Arg Val Glu Phe Ser Ala Tyr Thr Gly Leu Cys Glu Asp
                325                 330                 335

Val Ile Arg Pro Gln Leu Asp Glu Ala Ile Ala Gln Gly Tyr Leu Thr
            340                 345                 350

Glu Cys Ala Asp Tyr Trp Gln Ile Thr Glu His Gly Lys Leu Phe Leu
        355                 360                 365

Asn Ser Leu Leu Glu Leu Phe Leu Ala Glu
    370                 375